# EUROPÄISCHE PATENTSCHRIFT

(11) **EP 1 222 312 B1**
(45) Veröffentlichungstag und Bekanntmachung des Hinweises auf die Patenterteilung: **29.12.2004**
(21) Anmeldenummer: 00969294.8
(22) Anmeldetag: 26.09.2000
(51) Int. Cl.: C12Q 1/68

(54) **MULTIPLEX-PCR ZUM NACHWEIS VON EHEC-INFEKTIONEN**
MULTIPLEX PCR FOR DETECTING EHEC INFECTIONS
PCR MULTIPLEX POUR METTRE EN EVIDENCE DES INFECTIONS A EHEC

(30) Priorität: 28.09.1999 DE 19946296
(43) Veröffentlichungstag der Anmeldung: 17.07.2002
(73) Patentinhaber: Cytonet GmbH & Co. KG, 69469 Weinheim (DE)
(72) Erfinder: GUNZER, Florian, 30559 Hannover (DE); BELLIN, Tobias, 30966 Hemmingen (DE)
(74) Vertreter: Schrell, Andreas, Dr.
(86) Internationale Anmeldenummer: PCT/EP2000/009356
(87) Internationale Veröffentlichungsnummer: WO 2001/023607

(56) Entgegenhaltungen:
- EP-A- 0 864 657
- WO-A-97/46707
- DE-A- 19 721 825
- US-A- 5 475 098
- US-A- 5 652 102
- SHARMA V K ET AL.: "Semi-automated fluorogenic PCR assays (TaqMan) for rapid detection of Escherichia coli O157:H7 and other Shiga toxigenic E.coli" MOLECULAR AND CELLULAR PROBES, Bd. 13, August 1999 (1999-08), Seiten 291-302, XP001001372
- FAGAN P K ET AL.: "Detection of Shiga-like toxin (stx1 and stx2), intimin (eaeA), and enterohemorrhagic Escherichia coli (EHEC) hemolysin (EHEC hlyA) genes in animal feces by multiplex PCR" APPLIED AND ENVIRONMENTAL MICROBIOLOGY, Bd. 65, Nr. 2, Februar 1999 (1999-02), Seiten 868-872, XP001001377
- READ S C ET AL: "POLYMERASE CHAIN REACTION FOR DETECTION OF VEROCYTOTOXIGENIC ESCHERICHIA COLI ISOLATED FROM ANIMAL AND FOOD SOURCES" MOLECULAR AND CELLULAR PROBES,ACADEMIC PRESS, LONDON,GB, Bd. 6, Nr. 2, 1992, Seiten 153-161, XP000993359 ISSN: 0890-8508
- FRANCK S M ET AL.: "Multiplex PCR for enterotoxigenic, attaching and effacing, and shiga toxin-producing Escherichia coli strains from calves" JOURNAL OF CLINICAL MICROBIOLOGY, Bd. 36, Nr. 6, Juni 1998 (1998-06), Seiten 1795-1797, XP001001373
- GANNON V P J ET AL.: "Rapid and sensitive method for detection of Shiga-like toxin-producing Escherichia coli on ground beef using the polymerase chain reaction" APPLIED AND ENVIRONMENTAL MICROBIOLOGY, Bd. 58, Nr. 12, Dezember 1992 (1992-12), Seiten 3809-3815, XP001001444 in der Anmeldung erwähnt
- PATON A W ET AL: "DETECTION AND CHARACTERIZATION OF SHIGA TOXIGENIC ESCHERICHIA COLI BY USING MULTIPLEX PCR ASSAYS FOR STX1, STX2, EAEA, ENTEROHEMORRHAGIC E. COLI HLYA, RFBA, RFB0111, AND RFB0157" JOURNAL OF CLINICAL MICROBIOLOGY,US,WASHINGTON, DC, Bd. 36, Nr. 2, 1998, Seiten 598-602, XP000908789 ISSN: 0095-1137
- CHEN S ET AL.: "An automated fluorescent PCR method for detection of shiga toxin-producing Escherichia coli in foods" APPLIED AND ENVIRONMENTAL MICROBIOLOGY, Bd. 64, Nr. 11, November 1998 (1998-11), Seiten 4210-4216, XP001001375 in der Anmeldung erwähnt
- KIDO C ET AL: "Rapid and simple detection of PCR product DNA: a comparison between Southern hybridization and fluorescence polarization analysis" GENE,ELSEVIER BIOMEDICAL PRESS. AMSTERDAM,NL, Bd. 259, Nr. 1-2, 23. Dezember 2000 (2000-12-23), Seiten 123-127, XP004228429 ISSN: 0378-1119
- FRANKE S ET AL.: "Clonal relatedness of Shiga-like toxin-producing Escherichia coli O101 strains of human and porcine origin" JOURNAL OF CLINICAL MICROBIOLOGY, Bd. 33, Nr. 12, Dezember 1995 (1995-12), Seiten 3174-3178, XP001001374 in der Anmeldung erwähnt
- BELLIN T ET AL.: "Rapid detection of enterohemorrhagic Escherichia coli by real-time PCR with fluorescent hybridization probes" JOURNAL OF CLINICAL MICROBIOLOGY, Bd. 39, Nr. 1, Januar 2001 (2001-01), Seiten 370-374, XP000994590

## Beschreibung

Die vorliegende Erfindung entstammt dem diagnostischen Gebiet des Nachweises von hämorrhagischen Durchfallerkrankungen.

Entero-hämorrhagische E.coli-Erreger (EHEC) sind gefährliche Erreger von Durchfallerkrankungen, die sowohl durch Nahrungsmittel als auch durch Schmierinfektionen übertragen werden können. Entero-hämorrhagische E.coli Keime sind in der Lage, hochpotente Zytotoxine zu bilden. Dabei handelt es sich um Proteine, die grosse Ähnlichkeit mit dem Shiga-Toxin von Shigella dysenteriae Typ 1 besitzen und deshalb Shiga-Toxin 1 und Shiga-Toxin 2 genannt werden. Die dafür codierenden Gene für die jeweiligen Untereinheiten A und B werden mit stxA1 GenBank-Nummer M 19473) und stxB1 (GenBank-Nummer M19473) bzw. stxA2 (GenBank-Nummer X07865) und stxB2 (GenBank-Nummer X07865) bezeichnet. Pathogene E.coli Stämme können entweder eines der beiden Shiga-Toxin-Gene oder aber auch beide enthalten. Darüber hinaus können die Erreger weitere assoziierte Virulenz-Faktoren wie EHEC-Intimin, EHEC-Hämolysin, EHEC-Katalase, EHEC-Serinprotease sowie EHEC-Enterotoxin aufweisen.

Der diagnostische Nachweis von EHEC ist wegen der Übertragungswege und der mit etwa 10²-10³ Keimen geringen Infektionsdosis nicht nur bei akut Erkrankten wichtig, sondern auch, um eventuelle Ausscheider zu identifizieren oder andere Infektionsquellen ausfindig zu machen. Nach dem Stand der Technik werden EHEC-Infektionen auf mikrobiologischem Wege mit Sorbit McConkey Selektivagarplatten oder mit Hilfe von Toxin-ELISAs nachgewiesen. Darüber hinaus existieren PCR-Nachweismethoden, mit deren Hilfe Shiga-Toxin-Gensequenzen nachgewiesen werden können (z.B. Chen et al., Applied and Environmental Microbiology Vol. 64 No. 11, S. 4210-4116, 1998; Gannon et al., Applied and Environmental Microbiology, Vol. 58 No. 12, S. 3809-3815, 1992; Pierard et al., Journal of Clinical Microbiology Vol. 36, No.11, S. 3317-3322, ). In der Routinediagnostik werden gegenwärtig jedoch ausschließlich Verfahren angewendet, welche die für die B Untereinheit kodierende Sequenz amplifizieren.

Eine Sequenzanalyse der Shiga-Toxin-Gene verschiedener Isolate hat gezeigt, dass sich nicht nur die Primärsequenzen von Shiga-Toxin 1 und Shiga-Toxin 2 voneinander unterscheiden, sondern daß darüber hinaus auch unterschiedliche EHEC-Isolate, bei denen Shiga-Toxin 2 immunologisch nachgewiesen werden kann, hinsichtlich der Sequenz des entsprechenden Gens unterschiedliche Allele aufweisen. Somit sind nur solche Primer-Sequenzen zum diagnostischen Nachweis von Shiga-Toxin 2 geeignet, die gegen hochkonservierte Regionen innerhalb der Shiga-Toxin Gene gerichtet sind und somit die Sequenzen sämtlicher Allele detektieren können.

Zusätzlich zu den klassischen, durch bekannte Human-pathogene EHEC-Erreger verursachte Durchfallerkrankungen existieren andere Durchfallerkrankungen mit einem ähnlichen Krankheitsbild. Bei diesen Erkrankungen konnten ebenfalls entero-hämorrhagische Erreger als Ursache durch Tests mit Verozellkulturen diagnostiziert werden (Pierard et al., Lancet 338, S. 762, 1991; Thomas et al., Eur.J.Clin.Microbiol.Infect.Dis. 13, S. 1074-1076, 1994). Allerdings waren diese Infektionen nicht durch molekulare Methoden gemäß dem Stand der Technik zum Nachweis Human-pathogener EHEC-Infektionen nachweisbar (Pierard et al., J.Clin.Microbiol. 36, S. 3317-3322, 1998). Da entsprechende immunologische Tests nur begrenzte Spezifität besitzen, wurden aufwendige mikrobiologische Anreicherungsverfahren und daran anschließende molekulare Sequenzanalysen durchgeführt. Dabei konnten derartige Erreger als entero-hämorrhagische E.coli-Stämme identifiziert werden, die bisher lediglich als Shiga-Toxin-produzierende Schweine-pathogene Keime bekannt waren (Pierard et al., Lancet 338, S. 762, 1991); Thomas et al.. Eur.J.Clin.Microbiol.Infect.Dis. 13, S. 1074-1076, 1994). Die Sequenzen der Shiga-Toxin-Gene dieser Schweine-pathogenen Isolate unterscheiden sich deutlich von denen Human-pathogener Stämme und werden mit stx2ₑ bezeichnet (Weinstein et al., J.Bacteriol. 170, S. 4223-4230, 1988; Franke et al., Journal of Clinical Microbiology Vol. 33 No. 12, S. 3174-3178, 1995).

Somit existiert bislang kein einfaches immunologisches oder molekularbiologisches Verfahren, mit dessen Hilfe die verantwortlichen Erreger sämtlicher entero-hämorrhagischer Durchfallerkrankungen beim Menschen nachgewiesen werden können.

Aufgabe der vorliegenden Erfindung ist deshalb die Bereitstellung eines Verfahrens, mit dem sowohl Human-pathogene als auch Schweine-pathogene EHEC-Erreger durch eine einzige Nachweisreaktion zu identifizieren sind.

Diese Aufgabe wird durch eine Nukleinsäure-Amplifikationsreaktion zum Nachweis von klinisch relevanten EHEC-Infektionen gelöst, bei dem gleichzeitig stxA1 und stxA2 Sequenzen identifiziert werden können, die sowohl von Human-pathogenen als auch von Schweine-pathogenen Erregern stammen.

Gegenstand der Erfindung ist somit ein Verfahren, bei dem in einer Multiplex-Amplifikations-Reaktion zum Nachweis von klinisch relevanten EHEC-Infektionen sowohl stxA1- als auch stxA2-Sequenzen amplifiziert werden, welches insbesondere dadurch gekennzeichnet ist, dass sowohl Human-pathogene stxA2 Isoformen als auch Schweine-pathogene stxA2ₑ-Isoformen amplifiziert werden. Dabei bezieht sich der Begriff "Multiplex-Amplifikations-Reaktion" auf PCR-Verfahren, bei denen mindestens zwei verschiedene Primer-Paare verwendet werden, von denen ein Primer-Paar zur Amplifikation von stx1-Sequenzen und ein zweites Primer-Paar zur Amplifikation von stx2-Sequenzen verwendet wird. Der Begriff "Schweine-Pathogen" wird im Rahmen dieser Anmeldung für Erreger verwendet, die ein Shiga-Toxin Gen stx2ₑ (Weinstein et al., J.Bacteriol. 170, S. 4223-4230, 1988), GenBank-Nummer: M21534) aufweisen und primär beim Schwein die Ödemkrankheit auslösen, aber auch beim Menschen zu Durchfallerkrankungen und extraintestinalen Krankheitsmanifestationen führen können.

Besonders geeignet zur Durchführung des erfindungsgemässen Verfahrens haben sich Primer einer Länge von 17-25 Nukleotiden erwiesen, deren Sequenzen entweder identisch mit einer Sequenz gemäss SEQ ID NO 1-4 ist, deren Sequenzen kontinuierliche Teilsequenzen einer der Sequenzen gemäss SEQ ID NO 1-4 darstellen, oder bei denen eine Sequenz gemäß SEQ ID NO 1-4 eine kontinuierliche Teilsequenz des Primers bildet. Die Verwendung von einem, bevorzugt mehrerer, besonders bevorzugt aller, erfindungsgemässen Primer zum Nachweis von EHEC-Infektionen ist ebenfalls Gegenstand der Erfindung.

Als vorteilhaft haben sich somit Multiplex-Amplifikations-Reaktionen zum Nachweis von klinisch relevanten EHEC-Infektionen erwiesen, bei denen entweder ein, mehrere oder alle erfindungsgemäßen Primer verwendet werden.

In der Regel werden als Primer chemisch synthetisierte deoxy-Ribonukleotide verwendet. Allerdings können im Prinzip auch andere Nukleinsäuremoleküle oder deren Derivate wie beispielsweise PNA (Peptide Nucieic Acids) eingesetzt werden. Darüber hinaus können erfindungsgemäße Primer auch mit detektierbaren oder immobilisierbaren Molekülen konjugiert sein.

In einer speziellen Ausführungsform der erfindungsgemässen Verfahrens wird das Produkt der Amplifikation zur Erhöhung der Sensitivität des Assays zusätzlich durch Hybridisierung detektiert. Die dafür geeigneten Hybridisations-Sonden besitzen vorzugsweise eine Länge von 25-35 Nukleotiden. In der Regel werden ebenfalls chemisch synthetisierte deoxy-Ribonukleotide verwendet, die wahlweise durch andere Nukleinsäuremoleküle oder deren Derivate wie beispielsweise PNA (Peptide Nucleic Acids) ersetzt werden können. Definitionsgemäß sind diese Sonden mit einer detektierbaren Markierung konjugiert. Dabei kann es sich zum Beispiel um einen Fluoreszenzfarbstoff, ein Enzym, ein radioaktives Atom oder um eine massenspektrometrisch nachweisbare Gruppe handeln.

Ebenfalls Gegenstand der Erfindung sind deshalb Hybridisations-Sonden mit einer Sequenz oder Teilsequenz gemäß SEQ ID NO 5-8. Es hat sich jedoch als vorteilhaft erwiesen, wenn eine Hybridisations-Sonde eine Sequenz aufweist, die identisch oder komplementär zu einer Region des stxA1-Gens bzw. des stxA2-Gens ist, welche dem enzymatisch aktiven Zentrum der durch diese Gene kodierten Polypeptid-Kette entspricht. In der Sequenz von stxA2 befindet sich dieses aktive Zentrum an Nukleotidposition 803-805 (Jackson et al, J.Bacteriol. 172, S. 3346-3350, 1990). Hybridisations-Sonden mit einer Sequenz oder Teilsequenz gemäß SEQ ID NO 8 sind somit besonders bevorzugt.

In einer weiteren besonderen Ausführungsform werden die erfindungsgemäss erhaltenen Multiplex-Amplifikationsprodukte mit Hilfe von Fluoreszenz-Detektion nachgewiesen. Bei der Wahl eines geeigneten fluoreszierenden Agens kann dieses bereits zum PCR-Ansatz hinzugegeben werden, ohne die Effizienz der Amplifikation zu beeinträchtigen. Dies kann beispielsweise dadurch erfolgen, dass die PCR-Reaktion in Gegenwart einer fluoreszierenden Verbindung durchgeführt wird, die bei Bindung an doppelsträngige DNA-Moleküle und Anregung mit Licht einer geeigneten Wellenlänge fluoreszierende Signale emittiert (WO 97/46707):

Gegenstand der Erfindung ist somit auch ein Verfahren, bei dem die Multiplex-Amplifikationsprodukte mit Hilfe einer bei Bindung an doppelsträngige DNA fluoreszierenden Verbindung detektiert werden. Beispielsweise kann der Fluoreszenz-Farbstoff SybrGreen für ein derartiges Verfahren eingesetzt werden (WO 97/46714).

Gegenstand der vorliegenden Erfindung ist ferner ein Verfahren, bei dem die stx-Sequenzen mit Hilfe einer oder mehrerer Fluoreszenz-markierter Hybidisationssonden nachgewiesen werden. Dabei sind verschiedene Ausführungsformen, wie etwa die Verwendung von Molecular Beacons (WO 95/13399, US-Patent Nr. 5,118,801) oder sogenannte TaqMan-Probes (WO 96/34983) möglich.

Für den quantitativen Nachweis von Nukleinsäuren sind auch mit Fluoreszenzfarbstoffen markierte Hybridisierungssonden wie beispielsweise Oligonukleotide geeignet, deren Bindung an ein Nukleinsäure-Target nach dem Prinzip des Fluoreszenz-Resonanz-Elektronen-Transfers (FRET) detektiert werden kann (WO 97/46707). Dabei wird eine sogenannte Donor-Komponente, beispielsweise Fluorescein, mit Licht einer bestimmten Wellenlänge angeregt. Bei räumlicher Nähe zu einer geeigneten Akzeptorkomponente wie beispielsweise bestimmten Rhodamin-Derivaten erfolgt dann ein Resonanz-Energie-Transfer auf die Akzeptorkomponente, sodaß das Akzeptormolekül Licht einer bestimmten Emissionswellenlänge emittiert.

Die Markierungen der Hybridisations-Sonden können nach Standardmethoden am 5' Ende, am 3' Ende oder auch intern erfolgen. In einer bevorzugten Ausführungsform sind die verschiedenen Farbstoffe an zwei verschiedenene Hybridisierungssonden gebunden, die in räumlicher Nähe an die Target-Nukleinsäure hybridisieren können. Wenn bei dieser Ausführungsform beide Hybridisations-Sonden an die Target-DNA gebunden sind, befinden sich auch beide Kompenenten des FRET-Systems in räumlicher Nähe zueinander, sodaß Fluoreszenz-Resonanz-Energie-Transfer gemessen werden kann. Dadurch wird indirekt eine Quantifizierung der Target-DNA möglich.

Die zwei Oligonukleotid-Sonden können dabei an den gleichen Strang der Target-Nukleinsäure hybridisieren, wobei der eine Farbstoff sich vorzugsweise am 3'-terminalen Nukleotid der ersten Sonde befindet und der andere Farbstoff sich vorzugsweise am 5' terminalen Nukleotid der zweiten Sonde befindet, sodaß die Distanz zwischen beiden nur eine geringe Anzahl von Nukleotiden beträgt, wobei diese Anzahl zwischen 0 und 30 liegen kann. Bei Verwendung von Fluorescein in Kombination mit einem Rhodamin-Derivat wie beispielsweise LC-RED-640 oder LC-RED-705 (Roche Molecular Biochemicals) haben sich Abstände von 0-15, insbesondere 1-5 Nukleotiden und in vielen Fällen von einem Nukleotid als vorteilhaft herausgestellt. Unter Wahrung der Nukleotid-Abstände zwischen den Farbstoffkomponenten können auch Sonden verwendet werden, die nicht terminal, sondern intern mit einem der Farbstoffe konjugiert sind. Bei doppelsträngigen Target-Nukleinsäuren können auch Sonden eingesetzt werden, die an verschiedene Stränge des Targets binden, sofern ein bestimmter Nukleotidabstand von 0 bis 30 Nukleotiden zwischen den beiden Farbstoff-Komponenten eingehalten wird.

Als besonders vorteilhaft haben sich demnach erfindungsgemäße Verfahren herausgestellt, bei denen stxA1 und stxA2 mit Hilfe von Fluoreszenz-Resonanz-Energie-Transfer nachgewiesen wird. Gegenstand der Erfindung sind ebenfalls Hybridisations-Sonden mit einer Sequenz oder Teilsequenz gemäß SEQ ID NO 5-8 sowie Verfahren, in denen diese spezifischen Hybridisations-Sonden zum Nachweis von klinisch relevanten EHEC-Infektionen eingesetzt werden.

Eine spezifische Ausführungsform der Erfindung stellen somit auch Fluoreszenz-markierte Sonden-Paare entweder gemäß SEQ ID NO 5 und 6 oder gemäß SEQ ID NO 7 und 8 dar, welche vorteilhafterweise mit jeweils einer FRET-Donor-Komponente wie beispielsweise Fluorescein bzw. mit einer FRET-Akzeptor-Komponente wie beispielsweise Cy5, LC-RED-640, LC-RED-705 oder einem anderen Rhodamin-Derivat markiert sind. Entsprechend markierte Oligonukleotidkombinationen werden im Folgenden als "FRET-Paare" bezeichnet.

Als besonders vorteilhaft hat sich der Einsatz derartiger FRET-Paare zum Nachweis von Amplifikationsprodukten während oder nach einer Multiplex-Amplifikationsreaktion herausgestellt. In einer besonderen Ausführungsform kann einer der beiden Amplifikationsprimer zugleich mit einem der beiden eingesetzten Farbstoffe markiert sein und somit eine der beiden Komponenten des FRET beisteuern.

Der Einsatz von geeigneten FRET-Paaaren zur Detektion der Multiplex-Amplifikationsprodukte ermöglicht ein paralleles, sogenanntes "Real-Time Monitoring" von PCR-Reaktionen, wobei Daten zur Generierung des Amplifikationsprodukts in Abhängigkeit von der Zahl der durchlaufenen Reaktionszyklen ermittelt werden können. Dies geschieht in der Regel dadurch, dass aufgrund der Reaktions- und Temperaturbedingungen während des notwendigen Annealings der Amplifikationsprimer an die nachzuweisende Nukleinsäure auch die Oligonukleotide des FRET-Paares an die Target-Nukleinsäure hybridisieren und bei geeigneter Anregung ein entsprechend messbares Fluoreszenzsignal emittiert wird. Aufgrund der erhaltenen Daten kann somit die Menge der ursprünglich eingesetzten Target-Nukleinsäure quantitativ bestimmt werden.

In einer anderen, bevorzugten Ausführungsform erfolgt die Detektion der Multiplex-Amplifikationsprodukte nach Beendigung der Amplifikationsreaktion, wobei nach Hybridisierung des FRET-Paares an die zu detektierende Target-Nukleinsäure die Temperatur im Rahmen einer Schmelzkurvenanalyse kontinuierlich erhöht wird. Gleichzeitig wird die in Abhängigkeit von der Temperatur emittierte Fluoreszenz gemessen und auf diese Weise eine Schmelztemperatur bestimmt, bei dem das eingesetzte FRET-Paar nicht mehr an die nachzuweisende Sequenz hybridisiert. Bei einem Auftreten von Mismatches zwischen dem eingesetzten FRET-Paar und dem Amplifikationsprodukt wird der Schmelzpunkt signifikant erniedrigt. Auf diese Weise können mit einem FRET-Paar verschiedene Target-Nukleinsäuren identifiziert werden, deren Sequenzen sich voneinander geringfügig durch eine oder wenige Punktmutationen unterscheiden.

Erfindungsgemäß wird dieses Prinzip in einer Multiplex-Amplifikationsreaktion zum Nachweis von EHEC Infektionen angewandt, bei der ein interner Standard eingesetzt wird, welcher sich von der stxA1 oder der stxA2 Wildtyp-Sequenz (GenBank-Nummer X07865) nur in ein oder zwei Punktmutationen unterscheidet. Somit können amplifizierte Target-Nukleinsäure und amplifizierter interner Standard mithilfe einer Schmelzkurvenanalyse voneinander unterschieden werden.

Dabei wird der Standard vorzugsweise nur in geringen Mengen von etwa 100 Plasmidkopien (1,7·10⁻²² mol) eingesetzt, so dass ein positives Signal hinsichtlich der Amplifikation des internern Standards nicht nur anzeigt, dass die PCR im jeweiligen Ansatz nicht in irgendeiner Weise inhibiert worden ist, sondern auch eine Kontrolle hinsichtlich der Sensitivität der Reaktion darstellt.

Ein weiterer Aspekt des erfindungsgemäßen Verfahrens betrifft die Unterscheidung von Human-pathogenem stxA2 und Schweine-pathogenem stxA2ₑ mithilfe der beschriebenen Schmelzkurvenanalyse.

Die Verwendung von FRET Paaren gem. SEQ ID NO 5 und 6 oder 7 und 8 zur Bestimmung von Schmelzkurven oder zur Unterscheidung von Human-pathogenem stx und Schweine-pathogenem stx ist in diesem Zusammenhang ebenfalls Gegenstand der Erfindung. Dabei wird vozugsweise ein FRET-Paar gem. SEQ ID NO 7 und 8 verwendet.

Gegenstand der vorliegenden Erfindung sind darüber hinaus Kits, die verschiedene Reagenzien zur Durchführung der erfindungsgemäßen Verfahren enthalten. Derartige erfindungsgemäße Kits enthalten in der Regel Amplifikations-Primer zur Durchführung einer Multiplex-PCR gem. SEQ ID NO 1-4. Vorzugsweise können diese Kits zusätzlich auch Hybridisations-Sonden beispielsweise mit Sequenzen gemäß SEQ ID NO 5-8 enthalten.

Darüber hinaus können diese Kits erfindungsgemäss zusätzlich Primer und Hybridisations-Sonden zur Amplifikation von DNA einzelner oder mehrerer zusätzlicher EHEC Virulenz-Faktoren wie beispielsweise EHEC-Intimin, EHEC-Hämolysin, EHEC-Katalase, EHEC-Serinprotease sowie EHEC-Enterotoxin enthalten. Sämtliche erfindungsgemäßen Kits können schliesslich zusätzlich Reagenzien enthalten, die generell zur Durchführung von Nukleinsäure-Amplifikationsreaktionen geeignet sind. Vorzugsweise, aber nicht ausschliesslich handelt es sich dabei um spezielle Puffer. Taq-Polymerase und deoxy-Ribonukleotide.

### Kurzbeschreibung der Abbildungen:

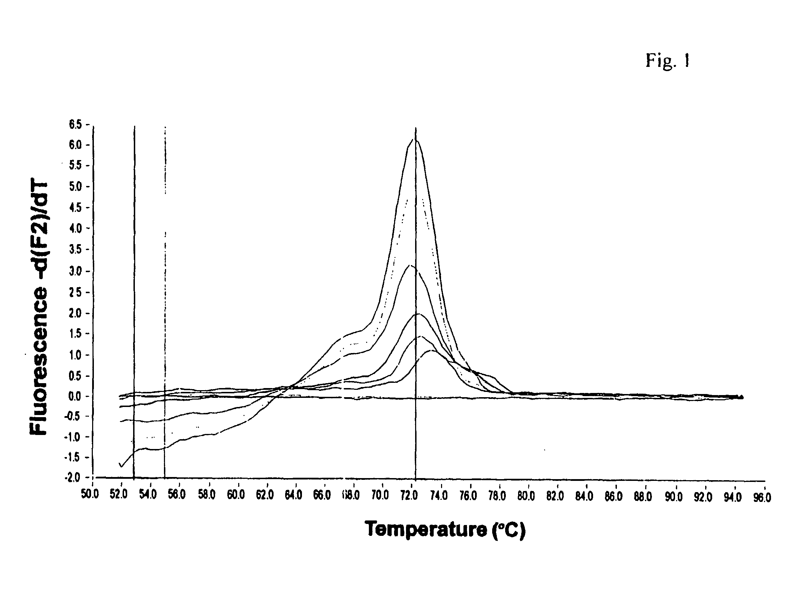

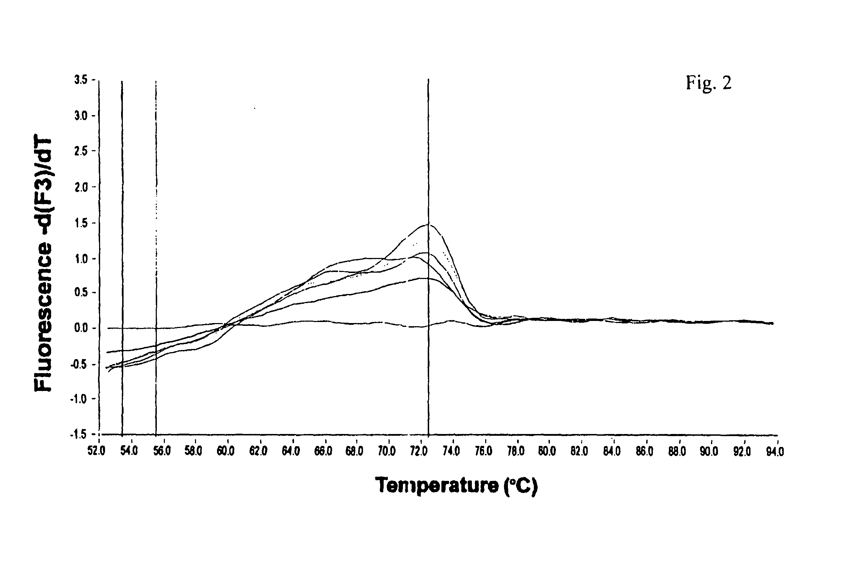

Abbildungen 1 und 2 zeigen eine Schmelzkurvenanalyse gemäß Beispiel 2. Dargestellt ist jeweils die erste Ableitung der gemessenen Fluoreszenz in Abhängigkeit von der jeweiligen Temperatur, gemessen mit einem FRET-Paar aus Fluorescein und LC-RED-640 zum Nachweis von stxA1 (Abbildung 1) und einem FRET-Paar aus Fluorescein und LC-RED-705 zum Nachweis von stxA2 (Abbildung 2).

### Beispiel 1: DNA-Isolierung aus Bakterienkulturen und Stuhlproben

Bakterienkulturen wurden nach Übernachtkultur in TSB-Bouillon (Caseinpepton, pankreatisch verdaut 17,0 g/l; Sojamehlpepton, papainisch verdaut 3.0 g/l; Natriumchlorid 5,0 g/l; Dikaliumhydrogenphosphat 2.5 g/l; Glucose 2,5 g/l.) mit Hilfe eines kommerziellen DNA-Extraktionsverfahrens (QIAamp DNA Mini Kit, Qiagen. Katalog Nr. 51304) aufgearbeitet. Dazu wurden 200 µl der Bakteriensuspension mit 20 µl Proteinase K und 200 µl Puffer ATL für 10 min bei 56°C inkubiert. Anschließed wurden der Suspension 200 µl 96 % Ethanol zugemischt. Die Lösung wurde dann auf eine QIAamp Spin-Säule gegben und für 1 min mit 6000 g abzentrifugiert. Danach wurden die Säulen einmal mit je 500 µl Puffer AW1 und AW2 gewaschen (Tischzentrifuge 20000 g). Nach dem zweiten Waschschritt wurde die Säule einmal trockenzentrifugiert. Die Elution der gereinigten DNA erfolgte im Anschluß daran mit 200 µl Puffer AE (10 mM Tris/HCC 0,5 m MEDT A pH 9.0). Vor der Verwendung in einer PCR-Reaktion wurden DNA-Konzentration und Reinheit in einem Photometer bestimmt (Spektrum von 260 nm bis 320 nm). Je PCR-Ansatz wurden maximal 500 ng Template-DNA eingesetzt. Bei Backgrounduntersuchungen wurd ein DNA-Äquivalent zu 10⁷ Bakterien (entspricht ca 55 ng DNA) eingesetzt.

Mit einem speziellen Stuhlkit (QIAamp DNA Stool Kit, enthaltend die gleichen Puffer wie der QIAamp DNA Mini Kit) wurden PCR-Untersuchungen direkt aus Stuhlproben durchgeführt. Dazu wurden 200 mg Stuhl (200 µl bei Durchfallstühlen) mit 600 µl Puffer ASL gründlich vermischt. Parallel dazu werden 300 mg der Matrix AX (Adsorbens für Inhibitoren in Stuhlproben) in 900 µl des gleichen Puffers resuspendiert. Diese Suspension wurde dann zu der gelösten Stuhlprobe gegeben und gründlich durchmischt. Anschließend wurde das Homogenat für 5 min bei 70°C inkubiert. Durch einen Zentrifugationsschritt von 3 min mit 20000 g wurden dann die Matrix AX und nicht gelöste Stuhlpartikel pelletiert. 200 µl des Überstandes wurden danach, analog zum o. g. QIAamp DNA Mini Protokoll mit 20 µl Proteinase K versetzt und, ebenso für 10 min bei 56°C inkubiert. Anschließend wurde die DNA ganz analog zu der Vorgehensweise für Bakterienkulturen, unter Verwendung der gleichen Puffer an eine QIAamp Spin-Säule gebunden, gwaschen, eluiert und im Photometer ausgemessen. Bei der anschließenden PCR-Reaktion wurden die gleichen DNA-Mengen, wie oben beschrieben, eingesetzt.

### Beispiel 2: Amplifikation und Identifizierung der Amplifikationsprodukte

Eine Multiplex-PCR zum Nachweis von stxA1 bzw stxA2 in DNA isoliert nach einem der Verfahren aus Beispiel 1 wurde im LightCycler System (Roche Molecular Biochemicals) entsprechend den Angaben des Herstellers durchgeführt. Der Nachweis des Amplifikationsproduktes erfolgte nach zwei unterschiedlichen Protokollen entweder mithilfe von SybrGreen als doppelsträngige DNA bindendes Agens oder alternativ mithilfe von FRET-Hybridisations-Sonden.

Sämtliche verwendeten Primer und Hybridisations-Sonden waren HPLC-gereinigt und wurden in Stocklösungen von 100 pM/µl (Primer) bzw. 3 pM/µl (Sonden) aufbewahrt. Die eingesetzten Primer wurden dabei so ausgewählt, daß von stxA1 ein 418 bp-Fragment (Nukleotidposition 598- 1015. Primer gemäß SEQ ID NO 1 und 2) und von stxA2 ein 264 bp-Fragment (Nukleotidposition 679-942, Primer gemäß SEQ ID NO 3 und 4) amplifiziert werden konnte.

Die zur Detektion eingesetzten Hybridisations-Sonden wurden nach Standardprotokollen markiert. Zum Nachweis von stxA1 wurde ein Oligonukleotid gemäß SEQ ID NO 5 mit Fluorescein am 3' Ende markiert und ein Oligonukleotid gemäß SEQ ID NO 6 am 5' Ende markiert. Als Hybridisations-Sonden für den Nachweis von stxA2 wurde ein Oligonukleotid gemäß SEQ ID NO 7 mit Fluorescein am 3' Ende und ein Oligonukleotid gemäß SEQ ID NO 8 mit LC-RED-705 am 5' Ende markiert.

| Nachweis mit SybrGreen: | |
|---|---|
| 2,0 µl | DNA-Master SYBR Green I (Roche Molecular Biochemicals, enthaltend Puffer, Taq DNA Polymerase, dNTPs, MgCl₂, und SYBR Green I Farbstoff) |
| 10 pM | je eingesetztem Primer gemäß SEQ ID NO 1-4 |
| 2,4 µl | 25 mM MgCl₂ (Arbeitskonzentration 4 mM) |
| 10 µl | DNA |
| 20 µl | Gesamtansatz |

| Nachweis mit Hybridisations-Sonden: | |
|---|---|
| 2.0 µl | DNA-Master for Hybridization Probes (Roche Molecular Biochemicals. enthaltend Puffer, Taq polymerase, dNTPs, and MgCl₂) |
| 10 pM | je eingesetztem Primer gemäß SEQ ID NO 1-4 |
| 3 pM | je eingesetzter Hybridisations-Sonde gemäß SEQ ID NO 5 und 6 für stxA1 der SEQ ID NO 7 und 8 stxA2 |
| 2,4 µl | 25 mM MgCl₂ (Endkonzentration 4 mM) |
| 8,0 µl | DNA |
| 20 µl | Gesamtansatz |

Beide Ansätze wurden mit dem gleichen PCR-Programm gefahren und mit einer Schmelzkurve abgeschlossen:

| Temperaturzyklen: | |
|---|---|
| 95°C | 120 sec Denaturierung zu Beginn des Programms |
| 95°C | 1 sec |
| 55°C | 5 sec (Touchdown von 60°C bis 55°C in 5 Schritten zu 1°C) |
| 72°C | 20 sec |
| 45 | Zyklen |

Die Schmelzkurven wurden nach vorheriger kurzzeitiger Denaturierung bei 95⁰C in einem Intervall von 50°C bis 95°C in 0,2°C-Schritten mit kontinuierlicher Fluoreszenzmessune auf Kanal 1 (SYBR Green), Kanal 2 (LC-Red 640) oder Kanal 3 (LC-Red 705) mithilfe der LightCycler Software 3.0 erstellt.

Im SYBR Green Modus wurde die Spezifität von PCR-Produkten aus Stuhlproben über den Schmelzpunkt im Vergleich zu einer Kontrolle aus stx-positiven Bakterien ermittelt, insbesondere um das Amplifikationsprodukt von unspezifischen Primer-Dimeren unterscheiden zu können. Darüber hinaus wurden alle Ergebnisse durch Gelelektrophorese verifiziert.

Im Falle des FRET-Hybridisations-Sonden-Formates war es erforderlich, den Color Compensation File der LightCycler 3.0 Software zur Vermeidung von Crosstalk-Effekten zwischen Kanal 2 und 3 einzusetzen. Die Ergebnisse eines typischen Experiments sind in Abbildung 1 und 2 offenbart:
Abbildung 1 zeigt die Temperatur-Abhängigkeit der Fluoreszenz aus Ansätzen mit unterschiedlichen DNA-Ausgangs-Konzentrationen in Kanal 2 (LC-RED-640) zum Nachweis von stxA1; Abbildung 2 zeigt die Fluoreszenz der gleichen Proben, gemessen in Kanal 3 (LC-RED-705) zum Nachweis von stxA2. Dargestellt ist jeweils die erste Ableitung des gemäß den Angaben des LightCycler-Herstellers gemessenen Fluoreszenzwertes in Abhängigkeit von der jeweiligen Schmelzkurventemperatur. Dadurch entsprechen die Temperaturwerte der ermittelten Kurven-Maxima den Schmelzpunkten der jeweiligen Hybridisations-Sonden.

### Beispiel 3: Sensitivität

DNA des stx1 und stx2- positiven E.coli-Stamms EDL 933 wurde gemäß Beispiel 1 extrahiert und photometrisch quantifiziert. Ausgehend von der Annahme, daß 2 x 10⁸ Bakterien ca. 1 µg DNA enthalten, wurde auf die Anzahl der aufgearbeiteten Bakterien rückgeschlossen und Verdünnungsreihen erstellt. Als Background wurden angezüchtete Stuhlproben aus der Routinediagnostik, die frei von darmpathogenen Erreger waren, mit der oben beschriebenen Methode bearbeitet. In Multiplex-Anätzen gemäß Beispiel 2 konnten dabei in einem Reaktionsansatz Äquivalente von mindestens ca. 1,8 stx-positiven Bakterien in einem Background von ca. 1 x 10⁷ stx-defizienten Bakteren nachgewiesen werden.

### Beispiel 4: Spezifität ― Nachweis von Human- und Schweine-pathogenen EHEC

48 humane Isolate unterschiedlichen Serotyps, deren Genotyp zum Zeitpunkt der Erfindung nicht bekannt war, die aber bereits durch andere Methoden nach dem Stand der Technik als EHEC-Stämme charkterisiert worden waren, wurden gemäß Beispiel 2 im FRET-Hybrisationssonden-Modus untersucht. Zusätzlich wurden 3 Isolate aus E.coli-Ödemerkrankten Schweinen untersucht. Das Ergebnis ist in Tabelle 1 dargestellt:

**Tabelle 1:**

| **Nachweis von Human- und Schweine-pathogenem stx** | | | | | | |
|---|---|---|---|---|---|---|
| Laufende-Nr. | Code-Nr. | Herkunft | Serotyp | *stx*_{*1*} | *stx*_{*2*} | Tₘ *stxA*_{*2*} |
| 1 | 485/98 | KI | O145:H⁻ | + | - | - |
| 2 | 531/98 | KI | O145:H⁻ | + | - | - |
| 3 | 563/98 | KI | O113:HNT | + | - | - |
| 4 | 633/98 | KI | O26:H⁻ | - | + | 72°C |
| 5 | 741/98 | KI | O121:H⁻ | - | + | 72°C |
| 6 | 742/98 | KI | O8:H⁻ | - | + | 63°C |
| 7 | 768/98 | KI | O30:H21 | - | + | 72°C |
| 8 | 802/98 | KI | O157:H⁻ | - | + | 72°C |
| 9 | 1115/98 | KI | O157:H⁻ | + | + | 72°C |
| 10 | 1168/98 | KI | O128:H⁻ | + | + | 63°C |
| 11 | 1211/98 | KI | O60:H⁻ | - | + | 63°C |
| 12 | 1244/98 | KI | O6:H8 | - | + | 72°C |
| 13 | 1273/98 | KI | O6:H8 | - | + | 72°C |
| 14 | 1295/98 | KI | ONT:H⁻ | - | + | 72°C |
| 15 | 1306/98 | KI | O157:H⁻ | + | + | 72°C |
| 16 | 1568/98 | KI | O103:H⁻ | + | - | - |
| 17 | 1613/98 | KI | ONT:HNT | + | - | - |
| 18 | 1695/98 | KI | O103:H⁻ | + | - | - |
| 19 | 1760/98 | KI | O129:H⁻ | + | + | 63°C |
| 20 | 1762/98 | KI | O129:H⁻ | + | + | 63°C |
| 21 | 1771/98 | KI | O113:H2 | + | + | 63°C |
| 22 | 54/99 | KI | O103:H18 | + | - | - |
| 23 | 90/99 | KI | O157:H⁻ | - | + | 72°C |
| 24 | 109/99 | KI | O157:H⁻ | - | + | 72°C |
| 25 | 143/99 | KI | O92:H32 | - | + | 63°C |
| 26 | 144/99 | KI | O92:H32 | - | + | 63°C |
| 27 | 159/99 | KI | O76:H⁻ | + | + | 63°C |
| 28 | 197/99 | KI | O128:HNT | + | + | 63°C |
| 29 | 209/99 | KI | ONT:HNT | - | + | 72°C |
| 30 | 240/99 | KI | O30:HNT | - | + | 72°C |
| 31 | 285/99 | KI | O145:HNT | + | - | - |
| 32 | 363/99 | KI | ONT:H⁻ | + | + | 63⁰C |
| 33 | 497/99 | KI | O103:H4 | + | - | - |
| 34 | 516/99 | KI | ONT:H⁻ | + | - | - |
| 35 | 575/99 | KI | O157:H⁻ | - | + | 72⁰C |
| 36 | 576/99 | KI | O157:H⁻ | - | + | 72⁰C |
| 37 | 594/99 | KI | ONT:H⁻ | + | - | - |
| 38 | 649/99 | KI | ONT:H9 | - | + | 72⁰C |
| 39 | 680/99 | KI | O157:H⁻ | + | + | 72⁰C |
| 40 | 707/99 | KI | ONT:HNT | + | + | 63⁰C |
| 41 | 713/99 | KI | O115:H10 | + | - | - |
| 42 | 720/99 | KI | O111:H⁻ | + | - | - |
| 43 | 789/99 | KI | O103:HNT | + | - | - |
| 44 | 791/99 | KI | O91:HNT | + | - | - |
| 45 | 809/99 | KI | ONT:HNT | + | - | - |
| 46 | 826/99 | KI | ONT:HNT | + | - | - |
| 47 | 827/99 | KI | ONT:HNT | - | + | 72⁰C |
| 48 | 834/99 | KI | ONT:HNT | + | - | - |
| 49 | A 3473-1/98 | OeK | O139:H1 | - | + | 63⁰C |
| 50 | A 3621-2/98 | OeK | O139:H1 | - | + | 63⁰C |
| 51 | 82812/99 | OeK | O139:H1 | - | + | 63⁰C |
| KI = klinisches Isolat | | | | | | |
| OeK = Ödemkrankheit | | | | | | |

Dabei konnten sämtliche humanen Isolate als stx positiv identifiziert werden, wobei in 18 Stämmen lediglich stx 1, in 19 Stämmen lediglich stx2 und in 11 Stämmen beide Gene nachgewiesen wurden. Die drei Schweine-Isolate waren ebenfalls stx2-positiv.

Bei Verwendung der erfindungsgemäßen Hybridisierungsproben gemäß SEQ ID NO 7 und 8 wurden bei verschiedenen Isolaten Unterschiede hinsichtlich der Schmelztemperaturen von stxA2 gemessen: Bei Amplifikaten aus 18 der 30 stxA2 enthaltenden humanen Isolate wurden für stxA2 Schmelztemperaturen von 71-72°C ermittelt. Diese Temperatur ist identisch mit dem auf Grund vorangegangener Experimente ermittelten Tm für klonierte stxA2 DNA aus Human-pathogenen Stämmen. Für die DNA der restlichen 12 stxA2 enthaltenden humanen Isolate sowie für die DNA aus den drei Schweine-pathogenen Stämmen, welche mit Sicherheit das stx2ₑ Allel enthalten, wurden Schmelztemperaturen von ca. 63°C ermittelt. Aus dem identischen Tm kann gefolgert werden, dass die 12 Human-pathogenen Isolate auf Schweine-pathogene EHEC Stämme zurückzuführen sind und vermutlich ebenfalls das stx2ₑ Allel enthalten. Diese Vermutung wurde durch Sequenzanalyse der PCR-Produkte aus den entsprechenden 12 Isolaten bestätigt.

Insgesamt zeigt dieses Beispiel, dass mit Hilfe des erfindungsgemäßen Verfahrens sowohl Human-pathogene als auch Schweine-pathogene EHEC Erreger identifiziert werden können.

### Beispiel 5: Spezifität - Vermeidung falsch positiver Ergebnisse

Spezifitätstests wurden an 32 in Tabelle 2 aufgelisteten stx-negativen Bakterienstämmen durchgeführt. Dazu wurde aus entsprechenden Übemachtkulturen DNA gemäß Beispiel 1 extrahiert. Anschließend wurde die isolierte DNA gemäß Beispiel 2 mithilfe des SybrGreen Modus auf die Präsenz von stxA1 und stxA2 untersucht. Das Ergebnis war immer eindeutig negativ. Als Inhibitionskontrolle wurde die DNA in einem paralellen Ansatz mit DNA des stx1 - und stx2 - positiven E. coli - Stammes EDL 933 vermischt und im gleichen Lauf auf stx1 und stx2 getestet, wobei ohne Ausnahme eindeutig positive Signale erhalten wurden.

**Tabelle 2:**

| **Im Spezifitätstes ausgetestete Bakterienisolate** | |
|---|---|
| *Aeromonas hydrophilia* | KLINISCHES ISOLAT |
| *Bacillus subtilis* | ATCC 6633 |
| *Bacillus subtilis* | ATCC 6051 |
| *Campylobacter coli* | KLINISCHES ISOLAT |
| *Campylobacter jejuni* | ATCC 33560 |
| *Candida albicans* | ATCC 10231 Typ 3 |
| *Citrobacter freundii* | KLINISCHES ISOLAT |
| *Enterobacter cloacae* | KLINISCHES ISOLAT |
| *Enterococcus faecalis* | ATCC 10541 |
| *Enterococcus faecalis* | KLINISCHES ISOLAT |
| *Enterococcus faecium* | ATCC 19434 |
| *Enterococcus faecium* | KLINISCHES ISOLAT |
| *Escherichia coli* | ATCC 25922 |
| EAEC O - 42 | KLINISCHES ISOLAT |
| EIEC 460858 | KLINISCHES ISOLAT |
| EPEC E 2348/69 | KLINISCHES ISOLAT |
| ETEC | KLINISCHES ISOLAT |
| *Helicobacter pylori* | KLINISCHES ISOLAT |
| *Klebsiella pneumoniae* | ATCC 10031 |
| *Morganella morganii* | KLINISCHES ISOLAT |
| *Plesiomonas shigelloides* | KLINISCHES ISOLAT |
| *Proteus mirabilis* | ATCC 1453 |
| *Proteus vulgaris* | KLINISCHES ISOLAT |
| *Pasteurella canis* | KLINISCHES ISOLAT |
| *Pseudomonas aeruginosa* | ATCC 27853 |
| *Salmonella enteritidis* | KLINISCHES ISOLAT |
| *Salmonella typhimurium* | KLINISCHES ISOLAT |
| *Shigella flexneri* | KLINISCHES ISOLAT |
| *Staphylococcus aureus* | KLINISCHES ISOLAT |
| *Staphylococcus epidermidis* | ATCC 12228 |
| *Streptococcus agalactiae* | KLINISCHES ISOLAT |
| *Yersinia enterocolitica* | KLINISCHES ISOLAT |

Somit zeigt dieses Beispiel, daß das erfindungsgemäße Verfahren zur spezifischen Detektion von EHEC Infektionen geeignet ist.

## Patentansprüche

1. Primer mit einer Länge bis zu 25 Nukleotiden umfassend die Nucleotidsequenz dargestellt in einer der SEQ ID NO. 1 bis 4.

2. Multiplex Amplifikationsreaktion zum Nachweis von klinisch relevanten EHEC-Infektionen, bei der sowohl stxA1 als auch stxA2 Sequenzen sowohl Human-pathogener als auch Schweine-pathogener stx2 Isoformen amplifiziert werden, **dadurch gekennzeichnet, dass** Primer mit der Nucleotidsequenz dargestellt in SEQ ID NO. 1 und Primer mit der Nucleotidsequenz dargestellt in SEQ ID NO. 2 und Primer mit der Nucleotidsequenz dargestellt in SEQ ID NO. 3 und Primer mit der Nucleotidsequenz dargestellt in SEQ ID NO. 4 verwendet werden.

3. Verwendung der Primer mit der Nucleotidsequenz dargestellt in SEQ ID NO. 1 und Primer mit der Nucleotidsequenz dargestellt in SEQ ID NO. 2 und Primer mit der Nucleotidsequenz dargestellt in SEQ ID NO. 3 und Primer mit der Nucleotidsequenz dargestellt in SEQ ID NO. 4 zum Nachweis einer EHEC Infektion.

4. Verfahren gemäß Anspruch 2, **dadurch gekennzeichnet, dass** das Produkt der Amplifikationsreaktion zusätzlich durch Hybridisierung detektiert wird.

5. Verfahren gemäß Anspruch 4, **dadurch gekennzeichnet, dass** mindestens eine Hybridisations-Sonde eine Nucleotidsequenz dargestellt in einer der SEQ ID NO. 5 bis 8 aufweist.

6. Verfahren gemäß Anspruch 2, 4 oder 5, **dadurch gekennzeichnet, dass** das Amplifikationsprodukt mithilfe von Fluoreszenzdetektion nachgewiesen wird.

7. Verfahren gemäß Anspruch 6, **dadurch gekennzeichnet, dass** das Amplifikationsprodukt mithilfe einer bei Bindung an doppelsträngige DNA fluoreszierenden Verbindung detektiert wird.

8. Verfahren gemäß Anspruch 4-6, **dadurch gekennzeichnet, dass** das Amplifikationsprodukt mithilfe von Fluoreszenz-Resonanz-Energietransfer nachgewiesen wird.

9. Verfahren gemäß Anspruch 8, bei dem ein interner Standard eingesetzt wird, welcher sich von der stxA1 oder der stxA2 Sequenz nur in ein oder zwei Punktmutationen unterscheidet, **dadurch gekennzeichnet, dass** amplifizierte Target-DNA und interner Standard mithilfe einer Schmelzkurvenanalyse voneinander unterschieden werden.

10. Verfahren nach Anspruch 8 oder 9, **dadurch gekennzeichnet**, das Human-pathogenes stxA2 und Schweine-pathogenes stxA2ₑ mithilfe einer Schmelzkurvenanalyse unterschieden werden.

11. Hybridisations-Sonde mit der Nucleotidsequenz dargestellt in einer der SEQ ID NO 5 bis 8.

12. Verfahren gemäß Anspruch 9 oder 10, **dadurch gekennzeichnet, dass** Hybridisations-Sonden mit Sequenzen gemäß Anspruch 11 verwendet werden.

13. Verwendung von Hybridisations-Sonden gemäß Anspruch 11 zur Bestimmung von Schmelzkurven.

14. Kit zum Nachweis klinisch relevanter EHEC-Infektionen, enthaltend Primer mit der Nucleotidsequenz dargestellt in SEQ ID NO. 1 und Primer mit der Nucleotidsequenz dargestellt in SEQ ID NO. 2 und Primer mit der Nucleotidsequenz dargestellt in SEQ ID NO. 3 und Primer mit der Nucleotidsequenz dargestellt in SEQ ID NO. 4.

15. Kit gemäß Anspruch 14 enthaltend Hybridisations-Sonden mit Sequenzen gemäß SEQ ID NO: 5-8.

16. Kit gemäß Anspruch 14 oder 15, enthaltend spezielle Püffer, Taq-Polymerase und deoxy-Ribonükleotide zur Dürchführung von Nükleinsaüre-Amplifikationsreaktionen.

## Claims

1. Primers with a length of up to 25 nucleotides comprising the nucleotide sequence shown in one of the SEQ ID No. 1 to 4.

2. Multiplex amplification reaction for detecting clinically relevant EHEC infections, in which both stxA1 and stxA2 sequences of both human-pathogenic and swine-pathogenic stx2 isoforms are amplified, **characterized in that** primers with the nucleotide sequence shown in SEQ ID No. 1 and primers with the nucleotide sequence shown in SEQ ID No. 2 and primers with the nucleotide sequence shown in SEQ ID No. 3 and primers with the nucleotide sequence shown in SEQ ID No. 4 are used.

3. Use of primers with the nucleotide sequence as shown in SEQ ID No. 1 and primers with the nucleotide sequence shown in SEQ ID No. 2 and primers with the nucleotide sequence shown in SEQ ID No. 3 and primers with the nucleotide sequence shown in SEQ ID No. 4 for detecting an EHEC infection.

4. Method according to claim 2, **characterized in that** the product of the amplification reaction is additionally detected by hybridization.

5. Method according to claim 4, **characterized in that** at least one hybridization probe has a nucleotide sequence shown in one of the SEQ ID No. 5 to 8.

6. Method according to claims 2, 4 or 5, **characterized in that** the amplification product is detected with the aid of fluorescence detection.

7. Method according to claim 6, **characterized in that** the amplification product is detected with the aid of a compound which fluoresces on binding to double-stranded DNA.

8. Method according to claims 4 to 6, **characterized in that** the amplification product is detected with the aid of fluorescence resonance energy transfer.

9. Method according to claim 8, in which an internal standard is used which differs from the stxA1 or the stxA2 sequence only in one or two point mutations, **characterized in that** amplified target DNA and internal standard are distinguished from one another by means of a melting curve analysis.

10. Method according to claim 8 or 9, **characterized in that** human-pathogenic stxA2 and swine-pathogenic stxA2ₑ are distinguished by means of a melting curve analysis.

11. Hybridization probe having a nucleotide sequence as shown in SEQ ID No. 5 to 8.

12. Method according to claim 9 or 10, **characterized in that** hybridization probes having sequences as given in claim 11 are used.

13. Use of hybridization probes according to claim 11 for determining melting curves.

14. Kit for detecting clinically relevant EHEC infections, comprising primers with the nucleotide sequence shown in SEQ ID No. 1 and primers with the nucleotide sequence shown in SEQ ID No. 2 and primers with the nucleotide sequence shown in SEQ ID No. 3 and primers with the nucleotide sequence shown in SEQ ID No. 4.

15. Kit according to claim 14, comprising hybridization probes having sequences according to SEQ ID No. 5 to 8.

16. Kit according to claim 14 or 15, comprising special buffers, Taq polymerase and deoxyribonucleotides for executing nucleic acid amplification reactions.

## Revendications

1. Amorce d'une longueur allant jusqu'à 25 nucléotides et comprenant la séquence de nucléotides représentée dans un des ID SEQ n° 1 à 4.

2. Réaction d'amplification multiplex pour détecter des infections par EHEC cliniquement pertinentes, dans laquelle aussi bien les séquences stxA1 que stxA2 d'isoformes stx2 tant pathogènes humaines que pathogènes de porc sont amplifiées, **caractérisée en ce que** l'on utilise des amorces ayant la séquence de nucléotides représentée dans l'ID SEQ n° 1 et des amorces ayant la séquence de nucléotides représentée dans l'ID SEQ n° 2 et des amorces ayant la séquence de nucléotides représentée dans l'ID SEQ n° 3 et des amorces ayant la séquence de nucléotides représentée dans l'ID SEQ n° 4.

3. Utilisation des amorces ayant la séquence de nucléotides représentée dans l'ID SEQ n° 1 et des amorces ayant la séquence de nucléotides représentée dans l'ID SEQ n° 2 et des amorces ayant la séquence de nucléotides représentée dans l'ID SEQ n° 3 et des amorces ayant la séquence de nucléotides représentée dans l'ID SEQ n° 4 pour détecter une infection par EHEC.

4. Procédé selon la revendication 2, **caractérisé en ce que** le produit de la réaction d'amplification est en outre détecté par hybridation.

5. Procédé selon la revendication 4, **caractérisé en ce qu'**au moins une sonde d'hybridation possède une séquence de nucléotides représentée dans l'un des ID SEQ n° 5 à 8.

6. Procédé selon la revendication 2, 4 ou 5, **caractérisé en ce que** le produit de l'amplification est détecté à l'aide d'une détection par fluorescence.

7. Procédé selon la revendication 6, **caractérisé en ce que** le produit de l'amplification est détecté à l'aide d'un composé qui est fluorescent en cas de liaison à de l'ADN bicaténaire.

8. Procédé selon la revendication 4 à 6, **caractérisé en ce que** le produit de l'amplification est détecté à l'aide d'une FRET (fluorescence-résonance-transfert d'énergie).

9. Procédé selon la revendication 8, dans lequel on utilise un étalon interne qui se distingue de la séquence stxA1 ou stxA2 uniquement par une ou deux mutations ponctuelles, **caractérisé en ce que** l'ADN cible amplifié et l'étalon interne sont différenciés l'un de l'autre à l'aide d'une analyse des courbes de fusion.

10. Procédé selon la revendication 8 ou 9, **caractérisé en ce que** stxA2 pathogène pour l'humain et stxA2ₑ pathogène pour le porc sont différenciés à l'aide d'une analyse des courbes de fusion.

11. Sonde d'hybridation ayant la séquence de nucléotides représentée dans un des ID SEQ n° 5 à 8.

12. Procédé selon la revendication 9 ou 10, **caractérisé en ce que** l'on utilise des sondes d'hybridation ayant des séquences selon la revendication 11.

13. Utilisation de sondes d'hybridation selon la revendication 11 pour déterminer des courbes de fusion.

14. Trousse de détection d'infections par EHEC cliniquement pertinentes, contenant des amorces ayant la séquence de nucléotides représentée dans l'ID SEQ n° 1 et des amorces ayant la séquence de nucléotides représentée dans l'ID SEQ n° 2 et des amorces ayant la séquence de nucléotides représentée dans l'ID SEQ n° 3 et des amorces ayant la séquence de nucléotides représentée dans l'ID SEQ n° 4.

15. Trousse selon la revendication 14 et contenant des sondes d'hybridation ayant des séquences selon les ID SEQ n° 5 à 8.

16. Trousse selon la revendication 14 ou 15, contenant des tampons spéciaux, de la Taq polymérase et des désoxyribonucléotides pour réaliser des réactions d'amplification des acides nucléiques.


### SEQUENZPROTOKOLL

<110> Roche Diagnostics Gmbh
<120> Multiplex-PCR zum Nachweis von EHEC-Infektionen
<130> 529800de
<140>
   <141>
<160> 8
<170> PatentIn Ver. 2.1
<210> 1
   <211> 24
   <212> DNA
   <213> Escherichia coli
<400> 1
   agtcgtacgg ggatgcagat aaat 24
<210> 2
   <211> 24
   <212> DNA
   <213> Escherichia coli
<400> 2
   ccggacacat agaaggaaac tcat 24
<210> 3
   <211> 20
   <212> DNA
   <213> Escherichia coli
<400> 3
   ttccggaatg caaatcagtc 20
<210> 4
   <211> 21
   <212> DNA
   <213> Escherichia coli
<400> 4
   cgatactccg gaagcacatt g 21
<210> 5
   <211> 30
   <212> DNA
   <213> Escherichia coli
<400> 5
   ctgtcacagt aacaaaccgt aacatcgctc 30
<210> 6
   <211> 24
   <212> DNA
   <213> Escherichia coli
<400> 6
   tgccacagac tgcgtcagtg aggt 24
<210> 7
   <211> 28
   <212> DNA
   <213> Escherichia coli
<400> 7
   agagcagttc tgcgttttgt cactgtca 28
<210> 8
   <211> 23
   <212> DNA
   <213> Escherichia coli
<400> 8
   agcagaagcc ttacgcttca ggc 23